# EUROPEAN PATENT APPLICATION

(11) **EP 1 995 218 A1**
(43) Date of publication of application: **26.11.2008**
(21) Application number: 06843239.2
(22) Date of filing: 26.12.2006
(51) Int. Cl.: C02F 1/04, B01D 3/36

(54) **METHOD OF TREATING PHENOL-CONTAINING WATER AND TREATING APPARATUS**

(30) Priority: 27.01.2006 JP 2006019785
(71) Applicant: Idemitsu Kosan Co., Ltd., Chiyoda-ku Tokyo 100-8321 (JP)
(72) Inventor: YOSHITOMI, Kazuyuki, Chiba 2990193 (JP); MASUDA, Shuichi, Chiba 2990193 (JP); KODAMA, Masahiro, Chiba 2990193 (JP)
(74) Representative: Vossius & Partner
(86) International application number: PCT/JP2006/325855
(87) International publication number: WO 2007/086224

(57) **Abstract**

The present invention relates to a method and an apparatus for treating phenol-containing water in which, in an azeotropic distillation column in which phenol-containing water is subjected to azeotropic distillation in the presence of an azeotropic agent for water, the phenol concentration in waste water is stably reduced to a low concentration without requiring a liquid-liquid extraction apparatus (extraction distillation column) and the like in the downstream side, by disposing an automatic gas chromatographic apparatus in a waste water line from an column top stationary separation-tank disposed on a top portion of the azeotropic distillation column and then by controlling the operation of the azeotropic distillation column by continuously measuring and monitoring the phenol concentration in the waste water by the automatic gas chromatographic apparatus and promptly detecting a change in the phenol concentration in the waste water.

## Description

### [Technical Field]

The preset invention relates to a method and an apparatus for treating phenol-containing water using an azeotropic distillation column. More particularly, the present invention relates to a method and an apparatus for treating phenol-containing water in which, in an azeotropic distillation column in which phenol-containing water is subjected to azeotropic distillation in the presence of an azeotropic agent for water, the phenol concentration in waste water is stably reduced to a low concentration by disposing an automatic gas chromatographic apparatus in a waste water line from a stationary separation tank disposed on a top portion of the azeotropic distillation column and then by controlling the operation of the azeotropic distillation column by continuously monitoring the phenol concentration in the waste water by the automatic gas chromatographic apparatus and promptly detecting a change in the phenol concentration in the waste water.

### [Background Art]

There is disclosed a method for treating a reaction product of phenol and acetone in which, in the method for treating a reaction product obtained by reacting phenol with acetone in the presence of an acid catalyst, the reaction product is separated into a column bottom product (I) consisting of unreacted phenol and bisphenol A and a column top product (I) consisting of unreacted phenol, unreacted acetone and water in the first distillation process, the column top product (I) is separated into a column bottom product (II) consisting of unreacted phenol and water and a column top product (II) consisting of acetone, and further the column bottom product (II) obtained in the second distillation process is separated into a column bottom product (III) consisting of phenol and a column top product (III) consisting of water in the third distillation process (for example, see Patent Document 1).
In addition, there is disclosed an effluent treatment method in which a phenol-containing waste water is subjected to azeotropic distillation in the presence of an azeotropic agent and then phenol is separated and recovered (For example, see Patent Document 2).
In a method for treating a mixture solution of phenol and water in the presence of an azeotropic agent for water using an azeotropic distillation column, the mixture solution is separated into a column bottom product consisting of phenol and a column top product consisting of water and the azeotropic agent, and the most important point to consider in the treatment method using the azeotropic distillation column is to prevent phenol from being discharged to the top portion of the column. If phenol is discharged to the top portion of the column, phenol is finally incorporated into the waste water. Generally, as the control of the azeotropic distillation column, it is common that the discharged composition is controlled by detecting the composition in the column by a thermometer installed in the column. However, when the concentration of phenol which is discharged to the top portion of the column and is to be controlled is in an order of ppm, it is extremely difficult to control the concentration of phenol discharged by a temperature at the top portion of the column.
Further, since a stationary separation tank (settler) for separating the azeotropic agent and water is installed at the column top portion in the azeotropic distillation column and a retention time of several tens of minutes is required to separate the azeotropic agent and water in the stationary separation tank, if once phenol is discharged to the top portion of the column and the phenol concentration in the stationary separation tank is increased, the waste water with a high concentration of phenol is discharged from the stationary separation tank for some time.

The phenol-containing waste water treated in the azeotropic distillation column is generally subjected to activated sludge treatment in an activated sludge tank installed within a factory before being discharged to sea or river. For this reason, if once the waste water with a high concentration of phenol is fed to the activated sludge tank, the activated sludge is immediately killed and the waste water is discharged without removal of phenol, thereby exerting an enormous influence on the environment.
In addition, this problem is not limited to a problem of the effluent treatment equipment such as an activated sludge tank, but the whole plant equipment or the whole factory in the worst case is in a state of shutdown, causing enormous damage. In order to avoid such situation, for the purpose of treating the waste water with a high phenol concentration, it is common to perform an operation in which a liquid-liquid extraction apparatus (extraction distillation column) is added in the downstream of the azeotropic distillation column, or once the phenol-containing waste water is stored in a waste water tank and then is transported to the activated sludge treatment equipment after confirming the phenol concentration in the waste water tank. Since these measures are not economical and the azeotropic distillation column is only changed for a brief period, when the phenol concentration in the waste water tank is increased and exceeds the activated sludge treatment receiving phenol concentration, the waste water in the waste water tank is required to be treated again by the azeotropic distillation column, causing a problem that the process becomes complicated.

Patent Document 1: Japanese Patent Laid-Open Publication No. H6-135874
Patent Document 2: Japanese Patent Laid-Open Publication No. 2000-107748

### [Disclosure of the Invention]

### [Problems to be Solved by the Invention]

The present invention is made to solve the above-described problems, and it is the object of the invention to provide a method and an apparatus for treating phenol-containing water in which, in an azeotropic distillation column in which phenol-containing water is subjected to azeotropic distillation in the presence of an azeotropic agent for water, the phenol concentration in the waste water is stably reduced to a low concentration without requiring a liquid-liquid extraction apparatus (extraction distillation column) and the like in the downstream side, by controlling the operation of the azeotropic distillation column by continuously monitoring the phenol concentration in the waste water discharged from a stationary separation tank disposed on a top portion of the azeotropic distillation column and promptly detecting a change in the phenol concentration in the waste water.

### [Means for Solving the Problems]

The present inventors have made earnest studies for attaining the above-mentioned object. As a result, the present inventors have found that the phenol concentration in the waste water may be stably reduced to a lower concentration by continuously monitoring the phenol concentration in the waste water discharged from a stationary separation tank disposed on a top portion of the azeotropic distillation column by an automatic gas chromatographic apparatus and then promptly detecting a change in the phenol concentration in the waste water, and have completed the present invention based on this finding.

That is, the present invention is:
(1) A method for treating phenol-containing water characterized in that, in an azeotropic distillation column in which phenol-containing water is subjected to azeotropic distillation in the presence of an azeotropic agent for water, the operation of the azeotropic distillation column is controlled by disposing an automatic gas chromatographic apparatus in a waste water line from a column-top stationary separation tank disposed on a top portion of the azeotropic distillation column and then continuously measuring and monitoring the phenol concentration in the waste water by the automatic gas chromatographic apparatus;
(2) The method for treating phenol-containing water described in the (1), wherein the azeotropic agent is at least one kind of azeotropic agent selected from xylene, toluene, benzene, cyclohexane, cumene and ethylbenzene;
(3) The method for treating phenol-containing water described in the (1), wherein a line which feeds a measurement sample to the automatic gas chromatographic apparatus is equipped with a coalescer;
(4) A treatment apparatus for phenol-containing water comprising an azeotropic distillation column and a column-top stationary separation tank disposed on a top portion of the azeotropic distillation column, wherein an automatic gas chromatographic apparatus is provided in a waste water line from the separation tank for determining the phenol concentration in the waste water; and
(5) further, the treatment apparatus for phenol-containing water described in the (4) comprising a coalescer in a line which feeds a measurement sample to the automatic gas chromatographic apparatus.

### [Brief Description of Drawings]

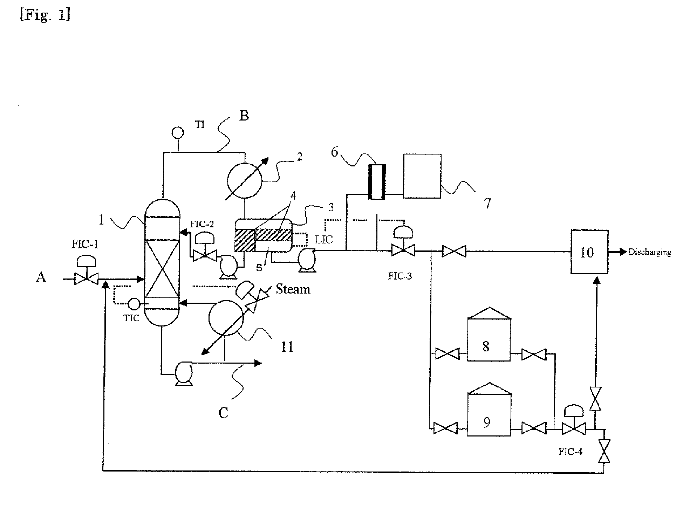

[Fig. 1] is a flowsheet showing an example of a treatment method for phenol-containing water of the present invention.

### [Explanation of Reference Numerals]

- 1:: Azeotropic distillation column
- 2:: Column top condenser
- 3:: Stationary separation tank
- 4:: Azeotropic agent
- 5:: Water
- 6:: Coalescer
- 7:: Automatic gas chromatographic apparatus
- 8:: Waste water tank
- 9:: Waste water tank
- 10:: Activated sludge treatment equipment
- 11:: Reboiler
- A:: Phenol-containing water
- B:: Mixed steam of azeotropic agent and water
- C:: Phenol
- FIC-1:: Automatic flow controller
- FIC-2:: Drawing flow controller
- FIC-3:: Reflux flow controller
- FIC-4:: Drawing flow controller
- TI:: Thermometer
- TIC:: Automatic temperature controller
- LIC:: Liquid level controller

### [Best Mode for Carrying Out the Invention]

The method for treating phenol-containing water of the present invention is characterized in that, in an azeotropic distillation column in which phenol-containing water is subjected to azeotropic distillation in the presence of an azeotropic agent for water, the operation of the azeotropic distillation column is controlled by disposing an automatic gas chromatographic apparatus in a waste water line from a column-top stationary separation tank disposed on the top portion of the azeotropic distillation column and then continuously measuring and monitoring the phenol concentration in the waste water by the automatic gas chromatographic apparatus.

The automatic gas chromatographic apparatus measuring phenol used in the present invention is a commonly used one, and is not particularly limited as long as it may set a separation column and an operation condition in which phenol may be analyzed in a ppm order.
Here, the automatic gas chromatographic apparatus refers to as an apparatus in which sampling and analysis are automatically performed without human hands.
Actually, in the following Examples and Comparative Example, the analysis was performed by the automatic gas chromatographic apparatus shown below. Specifically, the analysis was performed by Analyzer Type GC8A & Processor Type GC8P & FID (a flame ionizer detector) manufactured by YOKOGAWA ELECTRIC CORPORATION under the conditions in which the thermostatic chamber temperature was 105°C, nitrogen was used as a carrier gas and column model numbers used were YGC-5181G-1902 and YGC-5171B-1810.
In addition, a small amount of free azeotropic agent may be incorporated in a sampling solution transported to the automatic gas chromatographic apparatus. If the azeotropic agent is incorporated in the sampling solution, the free azeotropic agent may cause the analytical error of the automatic gas chromatographic apparatus. Specifically, if the droplets of the free azeotropic agent are fed to the automatic gas chromatographic apparatus, attention should be paid to the result that the phenol concentration is exceptionally high as the analytical result of the automatic gas chromatographic apparatus, because a large amount of phenol is contained in the free azeotropic agent, although the actual phenol concentration is not high. As mentioned above, since a serious problem occurs that the activated sludge is killed if the waste water with a high phenol concentration is treated by the activated sludge equipment, an operation is required to switch the transportation of the waste water to the waste water tank until it is found that there is no problems with the phenol concentration by performing the phenol analysis again by another manual gas chromatographic apparatus.

In order to avoid such problems, a coalescer is preferably disposed in a line which feeds a measurement sample to the automatic gas chromatographic apparatus. After the free azeotropic agent, which is incorporated in a small amount in the measurement sample, is removed by the coalescer disposed, the measurement sample may be fed to the automatic gas chromatographic apparatus, thereby enabling to prevent the occurrence of the analytical error due to the azeotropic agent in the automatic gas chromatographic apparatus. The azeotropic agent collected by the coalescer is returned to the stationary separation tank.
The coalescer is not particularly limited as long as it performs the function of collecting the free azeotropic agent, and includes, for example, a cartridge filter (1 micron, cellulose resin) manufactured by CUNO K.K.

The azeotropic agent for water used in the present invention includes at least one kind selected from xylene, toluene, benzene, cyclohexane and ethylbenzene, and preferred is ethylbenzene which is most economical.

The azeotropic distillation column used in the present invention is not particularly limited, and a distillation column, which is usually used for distillation, may be used as is. Further, there may be used either a packed type distillation column or a multi-stage tray type distillation column. As the typical operation conditions of the azeotropic distillation column, the operation is performed under the conditions in which the pressure in the column is approximately 70 to 80 kPa-A, the column bottom temperature is 160 to 70°C, the column top temperature is 80 to 90°C and the reflux ratio (R/D) is approximately 2 to 2.5.

There is performed a method for controlling the operation of the azeotropic distillation column by monitoring the automatic gas chromatographic apparatus which continuously measures the phenol concentration in the waste water in the present invention. The method is performed, for example, by the following procedure.
That is, a measurement sample is taken at an interval of 15 minutes and the phenol concentration is measured by the automatic gas chromatographic apparatus. When the upper limit of allowable concentration of phenol is set at 200 ppm by mass and it is detected that the phenol concentration measured at an interval of 15 minutes is 100 ppm which is approximately a half of the upper limit of allowable concentration, the operation is performed by raising the reflux ratio of the azeotropic distillation column by approximately 0.01 higher than the initial setting value and the subsequent transition of the phenol concentration is monitored. Even when phenol concentration is increased, the operation is performed by further raising the reflux ratio by approximately 0.01. In addition, when improvement is not observed by adjusting the reflux ratio, the operation is adjusted so that the setting value of the column bottom temperature of the azeotropic distillation column is decreased, or the feed phenol content is reduced.
By continuously analyzing the phenol concentration by the automatic gas chromatographic apparatus, monitoring the change of the detected values (analysis) measured by the automatic gas chromatographic apparatus and controlling the feed volume of the phenol-containing water to the azeotropic distillation column, the column bottom temperature, the reflux ratio and the like, in this manner, it becomes possible to treat the phenol-containing water in which the phenol concentration in the waste water is stably reduced to a low concentration without requiring a liquid-liquid extraction apparatus (extraction distillation column) and the like in the downstream side.

Next, there is explained the method for treating the phenol-containing water of the present invention according to the attached drawing.
Fig. 1 is an example of the method for treating the phenol-containing water of the present invention.
Firstly, the phenol-containing water A to be treated is fed to an azeotropic distillation column 1 through an automatic flow controller (FIC-1). In the azeotropic distillation column 1, the phenol-containing water A is heated by a reboiler 11 and the column bottom temperature is controlled at a predetermined temperature by an automatic temperature controller (TIC).
The mixed steam B of an azeotropic agent and water coming out from the column top of the azeotropic distillation column 1 is condensed by a column top condenser 2 to feed to a stationary separation tank 3 and then subjected to stationary separation into the azeotropic agent 4 and the water 5. The azeotropic agent 4 is refluxed to the upper portion of the azeotropic distillation column 1 at a predetermined reflux ratio (drawing flow controller: FIC-2/the reflux flow controller: FIC-3), and the water 5 is transported to waste water tanks 8 and 9 or an activated sludge treatment equipment 10 as waste water by a liquid level controller (LIC) of the stationary separation tank 3 and the drawing flow controller (FIC-2). On the other hand, phenol C is discharged from the column bottom of the azeotropic distillation column 1.
An automatic gas chromatographic apparatus 7 for automatically sampling a small volume of the waste water and continuously measuring the phenol concentration in the waste water is disposed in the waste water line. In addition, a coalescer 6 is preferably disposed in the line which feeds a sample to the automatic gas chromatographic apparatus 7.
If the phenol concentration in the waste water is a predetermined value or less, the waste water is directly fed to the activated sludge treatment equipment 10 as a normal line. However, if, for some reasons, the phenol concentration in the waste water measured by the automatic gas chromatographic apparatus exceeds a predetermined value, the waste water is once stored in the waste water tank 8 or 9. Subsequently, the phenol concentration in the waste water is measured again and when the phenol concentration actually exceeds the predetermined value, the waste water in the waste water tanks 8 and 9 is recycled to the azeotropic distillation column 1. On the other hand, if it may be confirmed that the phenol concentration is actually the predetermined value or less, the waste water in the waste water tanks 8 and 9 is fed to the activated sludge treatment equipment 10.

The present invention also provides a treatment apparatus for phenol-containing water comprising an azeotropic distillation column and a column-top stationary separation tank disposed on a top portion of the azeotropic distillation column, wherein an automatic gas chromatographic apparatus is provided in a waste water line from the separation tank for determining the phenol concentration in the waste water.
In the treatment equipment for phenol-containing water, the line which feeds a measurement sample to the automatic gas chromatographic apparatus is preferably equipped with a coalescer for the reason explained above.

### [Examples]

Next, the present invention is explained in more detail by Examples, but the present invention is not at all limited by these Examples.

### Example 1

Phenol-containing water (30% by mass of phenol and 70% by mass of water) was treated at a rate of 5 t/hr using ethylbenzene as an azeotropic agent in an azeotropic distillation column. The azeotropic distillation column was controlled under reduced pressure (70 kPa-A) by setting the column bottom temperature at 168°C. The column top temperature was 81 to 82°C.
Phenol is drawn from the column bottom at a rate of 1.5 t/hr, and ethylbenzene and water were drawn from the column top in the azeotropic composition, and then condensed by a column top condenser and subsequently subjected to stationary separation into ethylbenzene and water in a stationary separation tank. The water separated was transported to a waste water treatment equipment (activated sludge equipment) as waste water (3.5 t/hr, annual drainage volume: around 30,000 tons).
The phenol concentration of a sampling solution of the waste water from the waste water line was continuously analyzed through a coalescer (1 micron filter, cellulose resin) by an automatic gas chromatographic apparatus. The continuous operation was performed for one year while controlling the feed volume to the azeotropic distillation column, temperature, reflux ratio and the like by monitoring the change of the detected values (analysis) measured by the automatic gas chromatographic apparatus. As a result, the phenol concentration of the waste water could be maintained at 200 ppm by mass or less for one year and directly transported to the effluent treatment equipment without passing through the waste water tank. In addition, no abnormal values were found in the analytical values of the automatic gas chromatographic apparatus.

### Example 2

Phenol-containing water was treated by the azeotropic distillation column in the same manner as in Example 1 except that the coalescer was not disposed in the sampling line of the waste water from the drainage like. The operation was performed for one year. During the period, a peak with a high phenol concentration was observed around once in a month. In this case, an operation was performed in which the transportation of the waste water was switched from the activated sludge equipment (effluent treatment equipment) to the waste water tank until it is found that a sample is collected again and there was no problems with the phenol concentration by performing the phenol analysis by another manual gas chromatographic apparatus, and the time required was three hours.

### Comparative Example 1

Phenol-containing water was treated by the azeotropic distillation column in the same manner as in Example 1 except that the automatic gas chromatographic apparatus was not provided. The operation is performed invariably at a column top temperature of 81 to 82°C for one year. The waste water was once transported to the waste water tank, a tank solution was sampled and then analysis by a manual gas chromatographic apparatus was performed.
When the phenol concentration of the waste water was 200 ppm by mass or less which may be transported to the effluent treatment equipment, the waste water was transported to the effluent treatment equipment (activated sludge equipment), and in other cases, retreatment was performed by the azeotropic distillation column. The retreated volume was a tenth of the whole drainage volume for one year (approximately 3,000 tons), and the utility for treating the waste water accounted for approximately 4,000 tons/y in terms of steam (500 kcal/t-steam).

### [Industrial Applicability]

It is the object of the invention to provide a method and an apparatus for treating phenol-containing water in which, in an azeotropic distillation column in which phenol-containing water is subjected to azeotropic distillation in the presence of an azeotropic agent for water, the phenol concentration in waste water is stably reduced to a low concentration without requiring a liquid-liquid extraction apparatus (extraction distillation column) and the like in the downstream side, by monitoring the phenol concentration in the waste water discharged from a stationary separation tank disposed on a top portion of the azeotropic distillation column and then promptly detecting a change in the phenol concentration in the waste water.

## Claims

1. A method for treating phenol-containing water, wherein, in an azeotropic distillation column in which phenol-containing water is subjected to azeotropic distillation in the presence of an azeotropic agent for water, the operation of the azeotropic distillation column is controlled by disposing an automatic gas chromatographic apparatus in a waste water line from an column-top stationary separation tank disposed on a top portion of the azeotropic distillation column and then continuously measuring and monitoring the phenol concentration in the waste water by the automatic gas chromatographic apparatus.

2. The method for treating phenol-containing water according to claim 1, wherein the azeotropic agent is at least one kind of an azeotropic agent selected from xylene, toluene, benzene, cyclohexane, cumene and ethylbenzene.

3. The method for treating phenol-containing water according to claim 1, wherein a coalescer is disposed in a line which feeds a measurement sample to the automatic gas chromatographic apparatus.

4. A treatment apparatus for phenol-containing water comprising an azeotropic distillation column and a column-top stationary separation tank disposed on a top portion of the azeotropic distillation column, wherein an automatic gas chromatographic apparatus is provided in a waste water line from the separation tank for determining the phenol concentration in the waste water.

5. The treatment apparatus for treating phenol-containing water according to claim 4, further comprising a coalescer in a line which feeds a measurement sample to the automatic gas chromatographic apparatus.
